# EUROPEAN PATENT APPLICATION

(11) **EP 3 943 002 A1**
(43) Date of publication of application: **26.01.2022**
(21) Application number: 19920153.4
(22) Date of filing: 13.05.2019
(51) Int. Cl.: A61B 5/026

(54) **METHOD FOR CALCULATING INSTANTANEOUS WAVE-FREE RATIO BY MEANS OF PRESSURE SENSOR AND CONTRAST IMAGE**

(30) Priority: 19.03.2019 CN 201910206541
(71) Applicant: Suzhou Rainmed Medical Technology Co., Ltd., Suzhou, Jiangsu 215123 (CN)
(72) Inventor: HUO, Yunfei, Suzhou, Jiangsu 215123 (CN); LIU, Guangzhi, Suzhou, Jiangsu 215123 (CN); WU, Xingyun, Suzhou, Jiangsu 215123 (CN)
(74) Representative: Boult Wade Tennant LLP
(86) International application number: PCT/CN2019/086609
(87) International publication number: WO 2020/186611

(57) **Abstract**

Disclosed is a method for calculating an instantaneous wave-free ratio based on a pressure sensor and angiogram images, comprising: acquiring pressures at the coronary artery ostium of heart by a blood pressure sensor in real-time, and storing the pressure values in a data linked table, and the data linked table being indexed by time and the time and real-time pressure being saved in the form of key-value pairs; finding out corresponding datas from the data queue based on time index using the angiography time as an index value, taking an average value of four wave-free pressure values as a wave-free pressure value Pa; obtaining a time Tn of an end phase of a diastolic period, namely of a wave-free period within one cycle according to the time index within the cycle; obtaining a length L of a segment of a blood vessel through angiogram images of two body positions, and obtaining a blood flow velocity V; calculating a pressure drop ΔP and calculating a pressure Pd which at the distal end of the blood vessel as Pd=Pa-ΔP, and further obtaining the instantaneous wave-free ratio. The present disclosure can accurately obtain the pressure value and the blood flow velocity within one cardiac cycle, greatly improving the accuracy of iFR.

## Description

### TECHNICAL FIELD

The present disclosure relates to the field of coronary artery imaging evaluation, and in particular to, a method for calculating an instantaneous wave-free ratio based on a pressure sensor and angiogram images.

### BACKGROUND

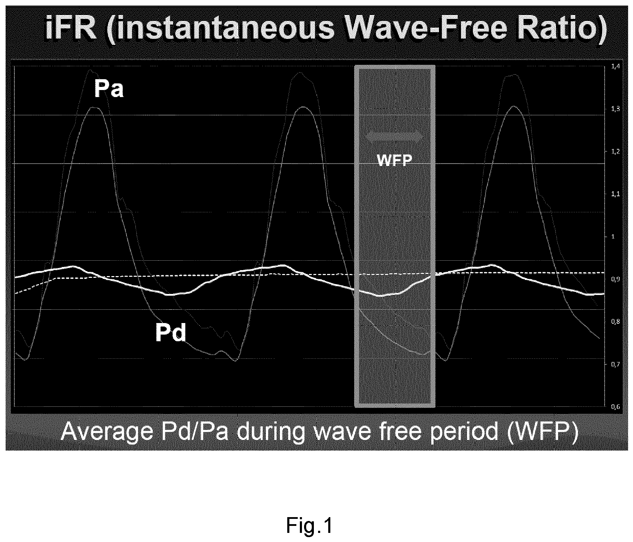

Instantaneous wave-free ratio (iFR) can provide a method of measuring intracoronary pressure similar to Fractional Flow Reserve (FFR). The iFR is free of using vasodilator and simple to operate, which will be used more extensively in coronary artery interventional therapy. The ADVISE's study found that during a certain period of time in the diastolic phase (called the wave-free period), the microvascular resistance in the coronary arteries is relatively the most stable and the lowest, which is similar to the average resistance presented during the period of coronary congestion made by using the adenosine or other vasodilators. As shown in Figure 1, iFR=P_{dWave-free} period/P_{aWave-free} period (P_{dWave-free period}: mean pressure in the distal coronary artery of the stenotic lesion during the wave-free period. Pawave-free period: mean pressure of the aorta during the wave-free period. Calculation time for an instantaneous wave-free period: calculating from 25% of the time after the start of the wave-free period during a diastolic period to 5 ms before the start of a systolic period).

The existing measuring method to get the instantaneous wave-free ratio (iFR) is to: measure the pressure values of the blood vessel at the end phase of diastole by the pressure guidewire in the resting state to determine the iFR. It is necessary to rely on the pressure guidewire for measurement, while this measurement process requires the pressure guidewire to be intervened into the end of the blood vessel, which is not only difficult, but also causes errors in the process of acquiring pressure, decreasing the accuracy of the calculated iFR.

### SUMMARY

In order to solve the above technical problems, the object of the present disclosure is to provide a method for calculating an instantaneous wave-free ratio based on a pressure sensor and angiogram images, which can accurately obtain a stable pressure value after administrating a contrast agent, then accurately obtain an average blood flow velocity within one cardiac cycle by the combination of a pressure waveform and the angiogram images, and thus can greatly improve the accuracy of iFR.

The technical solution of the present disclosure is:
a method for calculating an instantaneous wave-free ratio based on a pressure sensor and angiogram images, characterized in that, comprising the following steps:
S01: acquiring pressures at the coronary artery ostium of heart by a blood pressure sensor in real-time, and storing the pressure values in a data linked table, and the data linked table being indexed by time, and the time and real-time pressure being saved in the form of key-value pairs;
S02: obtaining an angiography time according to an angiogram image, by using the angiography time as an index value, finding out corresponding data from data queue based on time index, screening out stable pressure waveforms from multiple cycles, taking an average value of four wave-free pressure values as a wave-free pressure value Pa; obtaining a time Tn of an end phase of a diastolic period, namely of a wave-free period within one cycle according to the time index within the cycle;
S03: specifying, in an angiogram image of a body position, a first frame of a contrast agent flowing out of a catheter port and locating a last frame after the time Tn ; marking the position of the catheter port of the first frame as a start point of a blood vessel, and the position where the contrast agent flows to the farthest point on the last frame of the angiogram image as an end point of the blood vessel; then segmenting the segment of the blood vessel; obtaining the segment of the blood vessel from an angiogram image of another body position, and after three-dimensional synthesis of the two body positions, obtaining the true length L of this segment of the blood vessel, then obtaining a blood flow velocity V=L/Tn;
S04: calculating a pressure drop ΔP from the coronary artery ostium to the distal end of the coronary artery for the segment of the blood vessel in step S03 using an ostium blood flow velocity V, and calculating the pressure at the distal end of the blood vessel as Pd=Pa-ΔP, and further calculating the instantaneous wave-free ratio according to iFR=Pd/Pa.

In a preferred technical solution, said step S01 further comprises: obtaining, according to the time and real-time pressure value in the data linked table, peak pressure values, valley pressure values and wave-free pressure values for a range of points from a first point to a n^{th} point by comparative sorting algorithm, continuously recording the peak pressure values, the valley pressure values and the wave-free pressure values to form a queue indexed by time and corresponding to the peak pressure values, the valley pressure values and the wave-free pressure values, until completing the calculation of the n^{th} point, and then sequentially taking the next n points according to the time index from the data linked table stored for calculation, and so on.

In a preferred technical solution, it takes the process from one peak pressure value to the next as one cycle, an average value of four peak pressure values as a systolic pressure, an average value of four valley pressure values as a diastolic pressure, and an average time Tm of the four cycles as the time of one cycle.

In a preferred technical solution, the stable pressure waveforms in step S02 are waveforms in which relative differences of peak values of the waveforms in successive multiple cycles are within 4 mmHg.

Compared with the prior art, the advantages of the present disclosure comprise accurately obtaining the stable pressure value after administrating the contrast agent, and accurately obtaining the average blood flow velocity within one cardiac cycle by the combination of a pressure waveform and angiogram images, which can greatly improve the accuracy of iFR.

### BRIEF DESCRIPTION OF DRAWINGS

The present disclosure will be further described below with reference to the drawings and embodiments:
Figure 1 is a schematic diagram of an instantaneous wave-free ratio (iFR);
Figure 2 is a flow chart of a method for calculating the instantaneous wave-free ratio based on a pressure sensor and angiogram images according to the present disclosure.

### DETAILED DESCRIPTION OF PREFERRED EMBODIMENTS

In order to make the purposes, technical solutions and advantages of the disclosure clearer, the present disclosure will be described in detail below in conjunction with the specific embodiments and the corresponding accompanying drawings. It should be understood that these descriptions are only exemplary and are not intended to limit the scope of the present disclosure. In addition, it omits the explanations of well-known structures and technologies in the following description to avoid unnecessary confusion of the concept of the present disclosure.

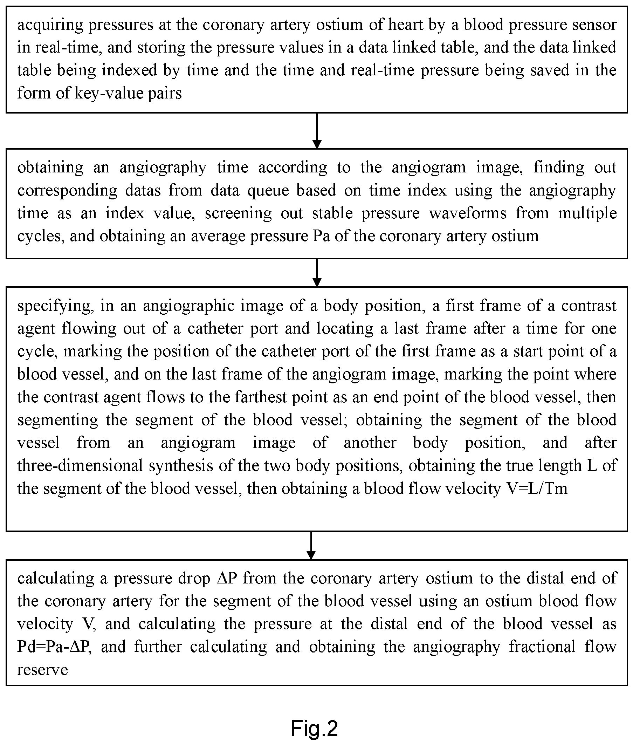

As shown in Fig. 2, the present disclosure tells a method for calculating the instantaneous wave-free ratio based on the pressure sensor and the angiography images, which comprises the following steps:
1. Pressures at the coronary artery ostium of heart are acquired by a blood pressure sensor in real-time, the pressure sensor communicating with the aorta through a pressure tube and a surgical catheter, which should keep the level of the pressure sensor and the heart being highly identical.
2. The pressure chip of the pressure sensor can sense the fluctuations of the pressures and generate an electrical signal, then the signal is transmitted to an acquisition chip of a control unit through a cable, and the electrical signal is converted by the acquisition chip into a pressure value and filtered to form a stable pressure waveform.
3. The pressure values are saved in a data linked table by a data processing chip of the control unit, and the data linked table being indexed by time, and the time and real-time pressures being saved in the form of key-value pairs.
4. There are n points counted from a first point according to the time and real-time pressure values in the data linked table by the data processing chip in which the number of n is based on the time index from the first point to a point at after at least 4 seconds, which is approximately 4 cardiac cycles or more. Peak pressure values, valley pressure values and wave-free pressure values in a range of points from the first point to the n^{th} point are obtained by comparative sorting algorithm (calculation time for a wave-free period: calculating from 25% of the time after the start of the wave-free period of a diastolic period to 5 ms before the start of a systolic period), the peak pressure values, the valley pressure values and the wave-free pressure values are continuously recorded to form a queue indexed by time and corresponding to the peak pressure values, the valley pressure values and the wave-free pressure values, until completing the calculation of the n^{th} point, and then sequentially taking the next n points according to the time index from the data linked table stored in step 3 for calculation, and so on.
5. Further, to calculate a systolic pressure, a diastolic pressure, the wave-free pressure and heart rate from the data queue saved in step 4, regarding one peak pressure value to the next as one cycle, taking an average value of four peak pressure values as the systolic pressure, taking an average value of four valley pressure values as the diastolic pressure, and taking an average value of four wave-free pressure values as the wave-free pressure value. The heart rate can be calculated by the average time Tm of the four cycles as 60/Tm. A time Tn of an end phase of a diastolic period, namely of the wave-free period within one cycle, is obtained according to the time index within the cycle; enabling parameters such as the systolic pressure, the diastolic pressure, the average pressure and the heart rate to be obtainable so as to provide more accurate data for the next steps.
6. As obtaining the pressure of the angiography blood vessel is needed for calculating iFR, which firstly needs to obtain the angiography time from an angiogram image, then finds out corresponding datas from the data queue in step 4 according to the time index using the angiography time as the index value, and later screens out stable pressure waveforms of 4 cycles from these datas in which the criterion for stable pressure is that relative differences of peak values of the waveforms of successive 4 cycles are within 4 mmHg. As it can obtain the wave-free pressure value Pa based on the method of step 5 and since the bolus injection of the contrast agent during the angiography will cause the pressure fluctuation to disappear, it can also accurately obtain the stable value for recovery of the pressure fluctuation after ceasing the bolus injection of the contrast agent according to the periodic data queue continuously recorded in step 4. Thus, it ensures that the acquired pressure physiological parameters of a patient are the corresponding parameters during the angiography.
7. Calculating the flow velocity is based on the heart rate obtained in step 6 and the time Tn of the wave-free period. A true length L of the segment of the blood vessel is obtained by three-dimensionally synthesizing two body positions, which, first in an angiogram image, specifies a first frame of the contrast agent flowing out of a catheter port and locates a last frame after the time Tn, marking the position of the catheter port of the first frame as a start point of the blood vessel, and the last frame where the contrast agent flowing to the farthest point as an end point of the blood vessel, and then segments the segment of the blood vessel, last equally obtains the segment of the blood vessel from an angiogram image of another body position. Thus the blood flow velocity can be obtained by V=L/Tn, which ensures that the average blood flow velocity within one cardiac cycle can be accurately obtained when calculating the flow velocity. As for the specific three-dimensional synthesis method, please refer to the Chinese patent document with an application number of 201610681191.1, which will not be repeated here in the present disclosure.
8. The value ΔP of the segment of the blood vessel in step 7 can be obtained by calculating the ostium blood flow velocity V through the computational fluid dynamics, wherein ΔP is a pressure drop from the coronary artery ostium to the distal end of coronary artery. As the pressure Pa which at the ostium of the blood vessel can be obtained through step 6, the pressure Pd which at the distal end of the blood vessel can be obtained as Pd=Pa-ΔP. Then the instantaneous wave-free ratio can be calculated by the instantaneous wave-free ratio calculation formula iFR=Pd/Pa.

As for the specific calculation methods of ΔP, please refer to the Chinese patent document with an application number of 201610681191.1, which will not be repeated here in the present disclosure.

It should be understood that the specific embodiments mentioned above are merely intended to exemplify or explain the principles of the present disclosure and not to be limitations to the present disclosure. Therefore, any modifications, equivalent substitutions, improvements and the like made without departing from the spirit and scope of the present disclosure should be included in the protection scope of the present disclosure. In addition, the appended claims of the present disclosure are intended to cover all the changes and modifications falling within the scope and boundary of the appended claims, or equivalents of such scope and boundary.

## Claims

1. A method for calculating an instantaneous wave-free ratio based on a pressure sensor and angiogram images, **characterized by** comprising the following steps:
S01: acquiring pressures at the coronary artery ostium of heart by a blood pressure sensor in real-time, and storing the pressure values in a data linked table, and the data linked table being indexed by time and the time and real-time pressure being saved in the form of key-value pairs;
S02: obtaining an angiography time according to an angiogram image, by using the angiography time as an index value, finding out corresponding datas from data queue based on time index, screening out stable pressure waveforms from multiple cycles, taking an average value of four wave-free pressure values as a wave-free pressure value Pa; obtaining a time Tn of an end phase of a diastolic period, namely of a wave-free period within one cycle according to the time index within the cycle;
S03: specifying, in an angiogram image of a body position, a first frame of a contrast agent flowing out of a catheter port and locating a last frame after the time Tn, marking the position of the catheter port of the first frame as a start point of a blood vessel, and the position where the contrast agent flows to the farthest point on the last frame of the angiogram image as an end point of the blood vessel; then segmenting the segment of the blood vessel; obtaining the segment of the blood vessel from an angiogram image of another body position, and after three-dimensional synthesis of the two body positions, obtaining the true length L of the segment of the blood vessel, then obtaining a blood flow velocity V=L/Tn;
S04: calculating a pressure drop ΔP from the coronary artery ostium to the distal end of the coronary artery for the segment of the blood vessel in step S03 using an ostium blood flow velocity V, and calculating the pressure Pd which at the distal end of the blood vessel as Pd=Pa-ΔP, and further calculating the instantaneous wave-free ratio according to iFR=Pd/Pa.

2. The method for calculating an instantaneous wave-free ratio based on a pressure sensor and angiogram images according to claim 1, **characterized in that** said step S01 further comprises: obtaining, according to the time and real-time pressure value in the data linked table, peak pressure values, valley pressure values and wave-free pressure values for a range of points from the first point to the n^{th} point by comparative sorting algorithm, continuously recording the peak pressure values, the valley pressure values and the wave-free pressure values to form a queue indexed by time and corresponding to the peak pressure values, the valley pressure values and the wave-free pressure values, until completing the calculation of the n^{th} point, and then sequentially taking the next n points according to the time index from the data linked table saved for calculation, and so on.

3. The method for calculating an instantaneous wave-free ratio based on a pressure sensor and angiogram images according to claim 1, **characterized in that** the process from one peak pressure value to the next is taken as one cycle, an average value of four peak pressure values as a systolic pressure, an average value of four valley pressure values as a diastolic pressure, and an average time Tm of the four cycles as the time of one cycle.

4. The method for calculating an instantaneous wave-free ratio based on a pressure sensor and angiogram images according to claim 1, **characterized in that** the stable pressure waveforms in step S02 are waveforms in which relative differences of peak values of the waveforms in successive multiple cycles are within 4 mmHg.
